# EUROPÄISCHE PATENTANMELDUNG

(11) **EP 0 670 304 A1**
(43) Veröffentlichungstag der Anmeldung: **06.09.1995**
(21) Anmeldenummer: 95102121.1
(22) Anmeldetag: 16.02.1995
(51) Int. Cl.: C07C 249/16, C07C 251/76

(54) **Verfahren zur Herstellung von substituierten Phenylhydrazonen**

(30) Priorität: 01.03.1994 DE 4406663
(71) Anmelder: MERCK PATENT GmbH, D-64271 Darmstadt (DE)
(72) Erfinder: Hopf, Martin, Dr., D-64846 Gross-Zimmern (DE); Sandner, Ingo, D-64287 Darmstadt (DE)

(57) **Zusammenfassung**

Die Erfindung betrifft ein verbessertes Verfahren zur Herstellung von substituierten Phenylhydrazonen ausgehend von Anilin-Derivaten und 1,3-Dicarbonylverbindungen, das dadurch gekennzeichnet ist, daß die Umsetzung des Diazonium-Salzes mit der 1,3-Dicarbonylverbindung in einem mit Wasser nicht mischbaren Lösungsmittel stattfindet sowie die Verwendung der so hergestellten Verbindungen zur Herstellung von Indolen.

## Beschreibung

Die Erfindung betrifft ein Verfahren zur Herstellung von substituierten Phenylhydrazonen aus einem Diazonium-Salz und einer 1,3-Dicarbonylverbindung. Substituierte Phenylhydrazone stellen wertvolle Zwischenprodukte zur Herstellung substituierter Indole dar.

Bisher wurden diese Verbindungen in einem zweistufigen Verfahren ausgehend von den entsprechenden Anilin-Derivaten hergestellt:
Das Anilin-Derivat wurde zuerst in ein Aniliniumhydrochlorid überführt, welches nach Isolierung mit einer wäßrigen Lösung von Natriumnitrit behandelt wurde. Anschließend wurde das so erhaltene Diazoniumsalz in einem wasserlöslichen Medium mit der 1,3-Dicarbonylverbindung zur Reaktion gebracht.

Setzt man jedoch das Aniliumhydrochlorid ohne vorherige Isolierung in der angegebenen Weise zur Herstellung der Phenylhydrazone ein, erhält man das Produkt in Form einer dunkelbraunen, schmierigen Masse, die auch nach Zusatz unpolarer Lösungsmittel nicht kristallisiert und nicht aufgearbeitet oder abgetrennt werden kann.

Aufgabe der vorliegenden Erfindung war es nun ein Verfahren aufzuzeigen, worin das Aniliniumhydrochlorid nicht in einer vorgeschalteten Reaktion hergestellt und isoliert werden muß, sondern direkt "in situ" hergestellt und weiterverarbeitet wird.

Überraschenderweise wurde nun gefunden, daß man auf die vorherige Isolation verzichten kann und dennoch zu kristallinen Produkten gelangt, wenn man die Umsetzung mit der 1,3-Dicarbonylverbindung nicht wie bisher in einem einphasigen protischen Lösungsmittel, sondern in einem Zweiphasen-System mit einem wasserunlöslichen Lösungsmittel durchführt.

Gegenstand der Erfindung ist somit ein Verfahren zur Herstellung von substituierten Phenylhydrazonen aus einem Diazonium-Salz und einer 1,3-Dicarbonylverbindung wobei man nacheinander folgende Schritte durchführt:
a) Umsetzen eines Anilin-Derivates mit Salzsäure,
b) Hinzufügen einer wäßrigen Natriumnitrit-Lösung,
c) Umsetzung mit der 1,3-Dicarbonylverbindung in einem inerten Lösungsmittel,
dadurch gekennzeichnet, daß man im Schritt c) ein mit Wasser nicht mischbares Lösungsmittel, vorzugsweise einen aromatischen Kohlenwasserstoff, insbesondere Toluol, verwendet.
a) Bevorzugte Ausführungsformen sind: Verfahren zur Herstellung von Phenylhydrazonen der Formel I 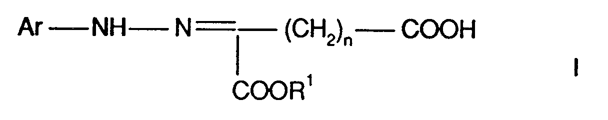 worin
   - Ar: eine unsubstituierte oder durch ein oder mehrere Alkyl oder Alkoxygruppen substituierte Phenylgruppe, vorzugsweise eine einfach durch C₁₋₁₀-Alkoxy substituierte Phenylgruppe,
   - R¹: eine C₁-C₈-Alkylgruppe, vorzugsweise C₁-C₄-Alkyl, und
   - n: 2, 3, 4 oder 5, vorzugsweise 2 oder 3, insbesondere 3, bedeuten,
   dadurch gekennzeichnet, daß man ein Anilin-Derivat der Formel II,

   Ar―NH₂ II

   worin Ar die angegebene Bedeutung besitzt,
   mit einem 2-Alkoxycarbonylcycloalkanon der Formel III, 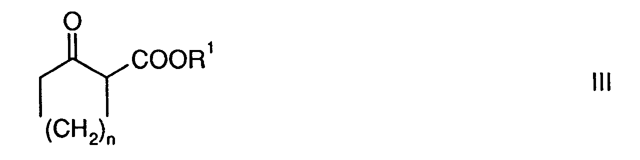 worin R¹ und n die angegebene Bedeutung besitzen, gemäß der Schritte a) bis c) umsetzt und d) anschließend mit einer Base, vorzugsweise einem Alkali- oder Erdalkalihydroxid, insbesondere mit konzentrierter Natronlauge behandelt.
b) Verfahren, wobei man bei Schritt c) einen pH zwischen 5,0 und 7,5 einstellt.
c) Verfahren, wobei man die Schritte a) bis c) in einer Apparatur ohne zwischenzeitliche Aufarbeitung und bei Temperaturen zwischen -20 °C und +25 °C durchführt.
d) Verfahren, wobei man bei Schritt d) die Base langsam so zudosiert, daß ein pH zwischen 9,0 und 4,0, vorzugsweise 8,0 und 5,0 eingehalten wird.
e) Verfahren, wobei man die Reaktion unter Rühren und Inertgasatmosphäre durchführt.

Ein weiterer Gegenstand der Erfindung ist die Verwendung der substituierten Phenylhydrazone, hergestellt nach einem der Ansprüche 1 bis 6 zur Herstellung von Indolen, dadurch gekennzeichnet, daß man sie in an sich bekannter Weise den Bedingungen einer Indolsynthese nach Fischer unterwirft.

Die Phenylhydrazone der Formel I sind wichtige Zwischenprodukte zur Herstellung von substituierten Indol-3-yl-alkylaminen, wertvollen pharmazeutischen Wirkstoffen mit blutdrucksenkenden, antidepressiv wirkenden Eigenschaften, insbesondere von Roxindol® (z.B. DE 28 27 874).

Die Durchführung des erfindungsgemäßen Verfahrens ist einfach.

Das Molverhältnis von Anilinderivat zu Salzsäure kann beliebig gewählt werden. In der Regel arbeitet man bei einem Molverhältnis von Anilinderivat zu Salzsäure im Bereich von 1:1,8 bis 1:4. Vorzugsweise beträgt dieses Verhältnis 1:2,0 bis 1:2,8.

Die Salzsäure wird in der Regel vorgelegt und das Anilin-Derivat hinzugefügt. Vorzugsweise wird die Salzsäure in Form einer 10 % bis 37%igen wäßrigen Lösung eingesetzt.

Zu der so erhaltenen wäßrigen Lösung des Aniliniumhydrochlorids wird eine wäßrige Natriumnitrit-Lösung hinzugefügt. Das Molverhältnis von Anilinderivat zu Natriumnitrit beträgt in der Regel 1:0,8 bis 1:1,2, vorzugsweise 1:0,9 bis 1:1,1, insbesondere 1:1,0 bis 1:1,05.

Die Natriumnitrit-Lösung wird vorzugsweise in Form einer 30 bis 60%igen Lösung eingesetzt.

Die Bildung des Diazoniumsalzes ist unter den angegebenen Bedingungen in der Regel nach 10 Minuten bis 120 Minuten abgeschlossen.

Die Zugabe des inerten, nicht mit Wasser mischbaren Lösungsmittels kann vor, während oder nach der Zugabe der 1,3-Dicarbonylverbindung erfolgen. Vorzugsweise fügt man die 1,3-Dicarbonylverbindung gelöst in dem inerten Lösungsmittel zu der wäßrigen Lösung des Diazoniumsalzes.

Das Molverhältnis von Anilin-Derivat zu 1,3-Dicarbonylverbindung wird vorzugsweise so gewählt, daß pro Mol eingesetztem Anilin-Derivat 0,8 bis 1,2, besonders bevorzugt 0,9 bis 1,1, insbesondere etwa 1,03 Mol 1,3-Dicarbonylverbindung eingesetzt werden.

Das Gewichtsverhältnis von 1,3-Dicarbonylverbindung zu dem inerten Lösungsmittel wird in der Regel so gewährt, daß pro Teil 1,3-Dicarbonylverbindung 2 bis 5, vorzugsweise 2,5 bis 3,5, insbesondere 3 bis 3,5 Teile Lösungsmittel eingesetzt werden.

Das erfindungsgemäße Verfahren kann man beispielsweise bei Temperaturen im Bereich von -25 bis +25 °C durchführen. Bevorzugte Temperaturen liegen im Bereich von -10 bis +10 °C, besonders bevorzugt im Bereich von -5 bis +5 °C. Das erfindungsgemäße Verfahren wird üblicherweise bei Normaldruck durchgeführt. Es kann jedoch auch bei vermindertem oder erhöhtem Druck durchgeführt werden. Die Anwendung von erhöhtem Druck ist insbesondere dann angezeigt, wenn man bei einer Reaktionstemperatur arbeiten möchte, bei der einzelne Bestandteile des Reaktionsgemisches bei Normaldruck sieden.

Geeignete Lösungsmittel sind Kohlenwasserstoffe, vorzugsweise aromatische Kohlenwasserstoffe, wie Benzol, Toluol oder Xylol, insbesondere Toluol.

Ar bedeutet in den Formeln I und II vorzugsweise eine durch ein bis vier, vorzugsweise eine oder zwei C₁-C₁₀-Alkyl oder Alkoxy substituierte Phenylgruppe.

In den genannten Verbindungen ist mindestens eine ortho-Position zum Stickstoffatom unsubstituiert, vorzugsweise weisen diese Verbindungen jedoch keinen Substituenten in ortho-Position zum Stickstoffatom auf.

Zur Herstellung der Verbindungen der Formel I wird in Schritt d) zu dem Gemisch eine Base zugesetzt.

Für das erfindungsgemäße Verfahren kommen in der Regel anorganische Basen, beispielsweise Alkali- und Erdalkalihydroxide in Frage. Besonders bevorzugt wird Natrium- oder Kaliumhydroxid verwendet.

Das Verhältnis von eingesetzter Dicarbonylverbindung zur Base wird vorzugsweise so gewählt, daß pro Äquivalent 1,3-Dicarbonylverbindung 0,9 bis 1,7, besonders bevorzugt 1,1 bis 1,5 Äquivalente Base eingesetzt werden.

Die Base wird vorzugsweise in Form einer 8 bis 32%igen wäßrigen Lösung zugesetzt. Die Zugabe erfolgt vorzugsweise unter pH-Wert Kontrolle. Am Ende der Reaktion wird ein schwach sauerer pH-Wert zwischen 4,0 und 6,0, insbesondere zwischen 5,0 und 5,5 eingestellt.

Die Aufarbeitung des Reaktionsgemisches ist unkritisch. Das Produkt kristallisiert nach 18 h aus, vorzugsweise wird nach 30 bis 48 h abfiltriert/geschleudert, mit Wasser und Toluol nachgewaschen und getrocknet.

Die Phenylhydrazone der Formel I werden in an sich bekannter Weise z.B. nach W.J. Houlihan, Ed., Chemistry of Heterocyclic Compounds-Indoles Pt 1, S. 232-316 (1972) durch Umsetzung mit Lewis-Säuren, insbesondere mit Zinkchlorid, in die Indole der Formel III überführt, 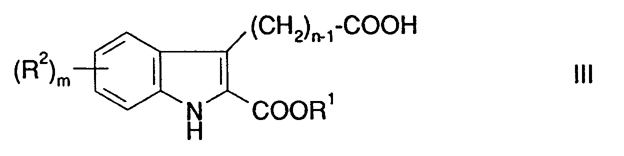
worin R¹ und n die angegebene Bedeutung besitzen,
- R²: Alkyl oder Alkoxy, und
- m: 0, 1, 2, 3 oder 4
bedeuten.

Nach dem erfindungsgemäßen Verfahren gelingt es überraschenderweise Phenylhydrazone ausgehend von Anilin-Derivaten und 1,3-Dicarbonylverbindungen in hohen Ausbeuten und großer Reinheit in einem Eintopfverfahren herzustellen. Das erfindungsgemäße Verfahren ist somit wesentlich wirtschaftlicher und erreicht im Vergleich zu dem Verfahren des Standes der Technik höhere Raum-Zeit-Ausbeuten.

### Beispiel 1

74,0 kg p-Anisidin werden unter Rühren und Kühlen zu einem Gemisch aus 138,2 kg konzentrierter Salzsäure und 395 l Wasser gegeben und zwei Stunden bei 0 °C unter Inertgasatmosphäre gerührt.

Anschließend fügt man innerhalb von 3 Stunden eine Lösung von 42 kg Natriumnitrit in 60 l Wasser bei Temperaturen zwischen 0 und +2 °C hinzu und läßt 30 Minuten weiter rühren.

Ein Gemisch aus 102 kg Cyclohexyl-2-carbonsäureethylester und 347 kg Toluol wird bei 0 °C innerhalb einer Stunde hinzugefügt und eine weitere Stunde gerührt.

Zu der erhaltenen Reaktionsmischung gibt man ein Gemisch aus 101 kg 32%iger Natronlauge und 248 l Wasser in etwa 4 Stunden bei Temperaturen unterhalb von 5 °C so zu, daß der pH-Wert von 8 nicht überschritten wird.

Nach Kristallation aus der Reaktionsmischung erhält man 136 kg 6-Carbethoxy-6-(4-methoxyphenylhydrazono)-hexancarbonsäure (≙ 70 % d. Th. bezogen auf p-Anisidin, Fp.: 97 °C).

### Vergleichsbeispiel

p-Anisidin wird wie in Beispiel 1 beschrieben umgesetzt, nur daß die Cyclohexan-2-carbonsäureethylester in Isopropanol anstelle von Toluol hinzugefügt wird.

Man erhält eine dunkelbraune, schmierige Masse, die - auch nach Zusatz von Toluol - nicht kristallisiert.

## Patentansprüche

1. Verfahren zur Herstellung von substituierten Phenylhydrazonen aus einem Diazonium-Salz und einer 1,3-Dicarbonylverbindung wobei man nacheinander folgende Schritte durchführt:
a) Umsetzen eines Anilin-Derivates mit Salzsäure,
b) Hinzufügen einer wäßrigen Natriumnitrit-Lösung,
c) Umsetzung mit der 1,3-Dicarbonylverbindung in einem inerten Lösungsmittel,
dadurch gekennzeichnet, daß man im Schritt c) ein mit Wasser nicht mischbares Lösungsmittel, vorzugsweise einen aromatischen Kohlenwasserstoff, verwendet.

2. Verfahren zur Herstellung von Phenylhydrazonen der Formel I 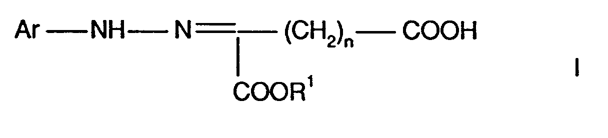 worin
Ar eine unsubstituierte oder durch ein oder mehrere Alkyl oder Alkoxygruppen substituierte Phenylgruppe,
R¹ eine C₁-C₈-Alkylgruppe, und
n 2, 3, 4 oder 5 bedeuten,
dadurch gekennzeichnet, daß man ein Anilin-Derivat der Formel II,
Ar―NH₂ II
worin Ar die angegebene Bedeutung besitzt,
mit einem 2-Alkoxycarbonylcycloalkanon der Formel III, 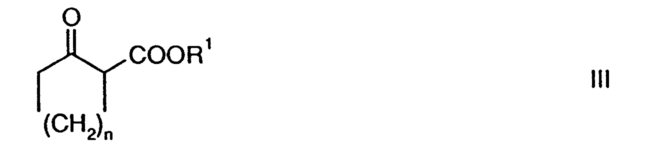 worin R¹ und n die angegebene Bedeutung besitzen, gemäß Anspruch 1 umsetzt und d) anschließend mit einer Base, vorzugsweise einem Alkali- oder Erdalkalihydroxid behandelt.

3. Verfahren nach einem der Ansprüche 1 oder 2, dadurch gekennzeichnet, daß man bei Schritt c) einen pH zwischen 5,0 und 7,5 einstellt.

4. Verfahren nach einem der vorangehenden Ansprüche, dadurch gekennzeichnet, daß man die Schritte a) bis c) in einer Apparatur ohne zwischenzeitliche Aufarbeitung und bei Temperaturen zwischen -20 °C und +25 °C durchführt.

5. Verfahren nach einem der Ansprüche 2, 3 oder 4, dadurch gekennzeichnet, daß man bei Schritt d) die Base langsam so zudosiert, daß ein pH zwischen 9,0 und 4,0, vorzugsweise 8,0 und 5,0 eingehalten wird.

6. Verfahren nach einem der vorangehenden Ansprüche, dadurch gekennzeichnet, daß man die Reaktion unter Rühren und Inertgasatmosphäre durchführt.

7. Verwendung der substituierten Phenylhydrazone, hergestellt nach einem der vorangehenden Ansprüche zur Herstellung von Indolen, dadurch gekennzeichnet, daß man sie in an sich bekannter Weise den Bedingungen einer Indolsynthese nach Fischer unterwirft.
